# EUROPEAN PATENT SPECIFICATION

(11) **EP 2 563 506 B1**
(45) Date of publication and mention of the grant of the patent: **08.09.2021**
(21) Application number: 11717195.9
(22) Date of filing: 21.04.2011
(51) Int. Cl.: B01J 10/00, B01J 19/00, C07C 273/04, B01J 19/02, B01J 19/24, B01D 3/00, B01D 3/14

(54) **REACTOR FOR UREA SYNTHESIS FROM AMMONIA AND CARBON DIOXIDE**
REAKTOR ZUR HARNSTOFFSYNTHESE AUS AMMONIAK UND KOHLENSTOFFDIOXID
RÉACTEUR POUR LA SYNTHÈSE D'URÉE À PARTIR D'AMMONIAC ET DE DIOXYDE DE CARBONE

(30) Priority: 30.04.2010 IT MI20100757
(43) Date of publication of application: 06.03.2013
(73) Proprietor: SAIPEM S.p.A., 20097 San Donato Milanese (MI) (IT)
(72) Inventor: CARLESSI, Lino, 24044 Dalmine (BG) (IT); GIANAZZA, Alessandro, I-20025 Legnano (Ml) (IT)
(74) Representative: Cernuzzi, Daniele
(86) International application number: PCT/EP2011/002106
(87) International publication number: WO 2011/134648

(56) References cited:
- EP-A1- 1 733 773
- AT-B- 90 360
- US-A- 3 364 124
- US-A- 3 634 471
- US-A- 4 582 569
- US-A- 6 165 315
- US-A1- 2002 190 403
- US-A1- 2006 032 620
- US-B1- 6 254 840
- None

## Description

The present invention relates to a reactor of the plate type for the production of urea by direct synthesis from ammonia and carbon dioxide in an environment comprising a liquid phase and a gaseous phase in contact with each other with a co-current flow, at a high pressure and temperature, with the intermediate formation of ammonium carbamate.

Unitary process operations on gas-liquid biphasic fluids mixed with each other, such as chemical reactions, evaporations, separations, etc., are normally carried out in vertical column equipment comprising a hollow cylindrical body, capable, if necessary, of tolerating high operating temperatures and pressures, closed at the ends by hemispherical caps, suitably shaped and equipped with the necessary openings for the outlet and inlet of the fluids, in which a plurality of perforated plates are arranged, suitably spaced, which define, in the cylindrical body, a series of geometrical cylindrical sectors in fluid communication with each other.

Reactors for the direct synthesis of urea starting from ammonia and carbon dioxide, with the formation of water as by-product, or for effecting inverse hydrolysis reactions of urea present in aqueous solutions destined for disposal, are of particular importance and difficult to construct. Both reactions are effected at high pressures and temperatures and require specific apparatuses for resisting saline corrosion due to the formation of ammonium carbamate as intermediate. These apparatuses operate at pressures normally ranging from 10 to 40 MPa and temperatures ranging from 70 to 300°C, in the presence of biphasic mixtures containing urea, water, ammonia, carbon dioxide and ammonium carbamate, the latter produced according to the known condensation reaction:

[2NH₃ + CO₂ + n H₂O → NH₄OCONH₂ • n H₂O]

The operating conditions are preferably a pressure of 12-25 MPa and a temperature of 120 to 240°C.

In normal industrial plants for the production of urea, the above high-pressure apparatuses generally contain volumes ranging from 10,000 to 400,000 liters. In order to obtain the maximum conversion, the two gas and liquid phases must be kept as dispersed as possible in each other, and back-mixing must be reduced to the minimum. For this purpose, the reactor normally consists of a vertical plate column whose height can even reach 60 meters, in plants have a capacity of over 2,000 tons/day. The plates inside the column are usually at a distance of 50 to 300 cm from each other and have various types of perforations in order to improve the re-mixing of the process fluids. Reactors equipped with numerous appliances and ducts for favouring fluid intercommunication between the sectors defined by each pair of adjacent plates, such as bells, grids, sleeves, overflows, etc., have also been described in the art. Among the numerous applications, the published patents EP 495418 and US 6,165,315 can be mentioned, for example.

In spite of the different solutions adopted in the processes currently in use, the considerable increase in the production of urea required by the market with the continuous expansion of its use as fertilizer, has led to the construction of column reactors with an increasingly high volume, in order to maintain adequate contact times and allow the maximum conversion to urea to be reached at thermodynamic equilibrium. The conversion of ammonium carbamate into urea by elimination of a water molecule is in fact a relatively slow step and requires that the reaction mixture be kept under suitable pressure and temperature conditions for times ranging from 15 to 120 minutes. In order to obtain these contact times with high volumes of reaction mixtures, the height of the reactor and number of plates must be increased, with the disadvantage of having to use excessively high reactors, characterized by a considerable encumbrance, difficulty in assembly and transportation, in addition to access to the internal parts for possible inspections and high pressure drops during functioning.

One of the solutions proposed is the use of two or more reactors per process line, which however have the disadvantage of increasing the construction, running and plant maintenance costs. It is not advantageous, on the other hand, to increase the volume of the reactor by widening the diameter of the column as this would lead to a considerable reduction in the conversion efficiency to urea, due to back-mixing phenomena and diffusion of the reaction mixture.

US6254840 discloses a gas-liquid contacting apparatus, in particular for use as a urea reactor for preparing urea from ammonia and carbon dioxide. The apparatus consists of a vertical column fitted with a series of perforated trays. Between adjacent trays is provided a generally cylindrical tube designed to increase the mixing effect between the liquid phase and the gas phase.

US2002/190403, US3364124 and US3634471 disclose different reactors/columns for biphasic operations, all having counter-current arrangements and elements designed to assist back-mixing of the different phases.

The need for effective and economical solutions which allow an increase in the production capacity of reactors for biphasic reactions, and in particular for the synthesis of urea, is therefore strongly felt.

The Applicant has now found that the above problems can be overcome by means of the reactor according to the present invention.

In accordance with this, the present invention firstly relates to a vertical reactor for the production of urea by direct synthesis from ammonia and carbon dioxide in a gas-liquid biphasic mixture.

A first object of the present invention therefore relates to a vertical reactor for the production of urea by direct synthesis from ammonia and carbon dioxide in a gas-liquid biphasic mixture for the production of urea by direct synthesis from ammonia and carbon dioxide in a gas-liquid biphasic mixtureas claimed in claim 1.

A second object of the present invention relates to a method for improving a pre-existing vertical reactor, of the plate type, for the production of urea by direct synthesis from ammonia and carbon dioxide in a gas-liquid biphasic mixture, as claimed in claim 13.

A further object of the present invention relates to a process according to claim 14 for the production of urea by direct synthesis starting from ammonia and carbon dioxide using the vertical reactor according to the present invention.

The term substantially cylindrical, as used herein, refers to any round structure similar to the form of the side surface of a cylinder, and also comprises, for example, structures having an elliptical or ovoidal section.

The term substantially hemispherical, as used herein, refers to any round structure similar to the shape of a hemisphere, and also comprises, for example, convex cap structures, with a non-uniform curvature radius.

The term vertical, as used herein, with reference to the reactor or its parts refers to the positioning of the apparatus during its normal functioning.

The term adjacent, as used herein, with reference to sectors or plates of the reactor, is meant in the longitudinal sense, i.e. meaning immediately above or immediately below in the vertical direction.

The term fluid communication, as used herein with reference to two hollow bodies, such as sections, sectors, cavities, means that at least one fluid, liquid or gas, is capable of flowing from one hollow body to the other during functioning.

The reactor according to the present invention can have various dimensions and be variably structured, depending on the reaction processes for which it is designed. The outer wall can have various thicknesses and consists of different metals, according to the pressures and temperatures of the process carried out in the reactor and in relation to the fluids involved. In the case of a reactor for the synthesis of urea, the normal pressures and temperatures are those previously indicated, and the fluids involved are highly corrosive under the process conditions, and consequently the construction materials consist of a force body made of steel having a considerable thickness, generally ranging from 50 to 300 mm, preferably from 100 to 250 mm, usually lined, internally, by one or more layers consisting of a corrosion-resistant metal such as, for example, AISI 316L steel (urea grade), INOX 25/22/2 Cr/Ni/Mo steel, special austenitic-ferritic steels, titanium, zirconium and relative alloys.

The diameter of the reactor according to the present invention can vary within wide limits and normally ranges from 50 to 500 cm. Preferred diameters for the application to the synthesis of urea range from 150 to 400 cm.

Two hemispherical caps (heads) are positioned at the ends of the cylindrical body of the reactor, in order to provide a better distribution of the pressure thrust. Openings for the inlet and outlet of the fluids, the introduction of possible sensors and an opening for inspections (manhole) are suitably situated inside the hemispherical caps and along the cylindrical body.

A variety of perforated plates is present inside the reactor, extending horizontally and perpendicular or substantially perpendicular to the main axis, according to the usual construction techniques of reactors for gas-liquid biphasic reactions, as is well illustrated, in particular for the formation of urea, in the documents previously cited. Said plates extend over the whole horizontal section of the reactor.

Each plate is equipped with a number of holes, which can be simple holes or comprise valves or bells, suitable for regulating the flow and dispersion of gas and liquid and their mixing and reciprocal contact. An average density of the holes is preferably present, ranging from 100 to 1,000 holes per m², with a distribution on the surface of the plate which can be more or less uniform, depending on the particular conditions of use. Furthermore, the density of the holes can also vary significantly from one plate to another in the same reactor, in accordance with the normal construction techniques, depending on the rheological characteristics and desired flows of the gas and liquid phases in the different areas of the reactor.

According to the present invention, the reactor for gas-liquid biphasic reactions is equipped in such a way as to allow co-current upward flowing of the gas and the liquid phases, mixed together. The height of the reactor is selected so as to obtain the desired contact time for bringing the reaction to equilibrium as much as possible. The number of plates is consequently selected so that the distance between one plate and the adjacent plate allows a good dispersion of the phases in each other to be maintained, so as to increase the exchange surface, but reduce or totally prevent back-mixing, which negatively influences reaching equilibrium. The preferred conditions are such as to obtain within the sector the so-called plug-flow (see reference Perry's Chemical Engineering Handbook, 5th ed., McGraw-Hill, page 5-41). In order to obtain these conditions the vertical reactor of the present invention do not usually comprise downcomers in the sectors between adjacent plates.

As the reactor is used for the synthesis of urea, the plates are at a distance of 50 to 400 cm from each other, preferably from 150 to 300 cm, with distances which conveniently increase with an increase in the diameter. Under these conditions, the number of plates present in the reactor varies from 5 to 40, preferably from 10 to 20. More preferably, the distance between adjacent plates is kept more or less constant along the whole reactor, except for a tolerance within +/-10% for construction requirements.

The plates are generally metallic, hinged onto the internal surface of the reactor by means of welding or preferably bolted, so that they can be more easily removed and substituted. The metal of which the plates is composed must have good resistance characteristics and hardness under the operating conditions of the reactor, particularly if corrosive fluids or rheological conditions which induce erosion phenomena, are present. For the synthesis of urea, the plates preferably consist of metals or alloys resistant to the corrosion of ammonium carbamate under the process conditions, for example, titanium, zirconium, AISI 316L steel (urea grade), special austenitic-ferritic steels and preferably INOX 25/22/2 Cr/Ni/Mo steel.

In accordance with the present invention, each section obtained by the interpositioning of a single dividing septum in a sector conveniently comprises a portion of the surface of both the upper and the lower plate in which there is at least one hole for fluid communication with the adjacent sector. The average density of the holes preferably ranges from 100 to 1,000, more preferably from 200 to 600, holes/m².

In the upper part, generally at the height of the cap, the reactor according to the present invention can be equipped with particular structures and additional parts, such as, for example, an opening for the upward outlet of part or all of the residual gaseous phase, collected through a suitable pipeline, in addition to an opening for collecting the liquid phase and instruments for controlling the temperature and pressure level.

As the reactor is designed for the synthesis of urea, the gas-liquid mixture comprising the reaction products and the residual part of non-converted reagents is preferably collected by means of an overflow situated close to the upper cap (head) of the reactor, and sent into a duct which descends vertically externally, or more preferably, internally, parallel to the axis of the reactor itself, until it reaches the height of the separation and purification apparatus of urea, in particular the stripper of the high-pressure synthesis cycle, whose top is at a height which is 0.5 to 0.8 times that of the reactor. This expedient, well-known to experts in the field, advantageously allows the pressure drop, due to the vertical development of the reactor, to be partly recovered.

According to the present invention, at least one, preferably at least two, more preferably at least 90% and even more preferably all, of said sectors delimited between two adjacent plates are divided into at least two sections by means of at least one dividing septum perpendicular, or substantially perpendicular, to said plates, preferably, but not necessarily, consisting of the same material as the plates themselves. The septa for the vertical reactor of the present invention are planar or composed by planar elements joined together, possibly forming a vertical angle. The term planar is herein intended as referreing to the overall size and shape of the septa or the elements thereof, and does not exclude the septa from being microstructured in various ways like being corrugated.

This septum can be of the closed type or open type. In the former case, the septum of the closed type does not allow the partial exchange of material between the sections into which the sector is divided. For this purpose, it is generally seal welded along the margins in contact with the plates and with the internal surface of the cylindrical body. In the latter case, although the septum is fixed to the plates and/or to the internal surface of the reactor by partial welding or bolting of the margins, it has one or more openings of fluid communication between the sections that it delimits. These openings can, for example, be close to the outer margins of each septum, along fixing lines, or they can consist of holes specifically created for this purpose in the septum. Both solutions are included in the scope of the present invention and allow the characteristics of the reactor to be optimally adapted to the kinetic and fluid-dynamic parameters of the reaction mixture. Dividing septa fixed in the reactor by bolting are preferred for their greater construction and maintenance simplicity.

The dividing septum is planar or substantially planar, and is preferably positioned so that its surface is close to or coincides with the main axis of the reactor, so as to divide the sector in which it is positioned into sections having a volume approximately equal to each other, in particular, with a ratio between the volumes of any two of the sections in which the sector is divided, ranging from 1.05 to 0.95. In accordance with this, when various dividing septa are present in a sector, for example two, they are preferably fixed by intersecting them so as to form dihedral angles, for example, four, and divide the sector into corresponding angular sections, more preferably having approximately the same volume.

According to the present invention, between two adjacent plates there is only a single dividing septum, more preferably positioned so as to intercept the axis of the reactor, i.e. oriented on one of the diameters of the adjacent plates. In this latter way, each sector is divided into two sections substantially having the same volume and geometry.

When said internal vertical duct for sending the reacted mixture to the separation unit is present, in the upper part of the reactor, it is conveniently positioned close to the internal wall of the reactor and crosses only one of the sections into which the sector is divided.

In accordance with the present invention, each of the dividing septa present in the reactor can be positioned with a directrix of the plane independent of the others. Should the sectors contain a single dividing septum, however, it has been found that two alternative solutions to each other are particularly advantageous. In the first of these solutions, all the dividing septa are substantially aligned on a single geometrical plane (except for insignificant deviations in the construction) containing the main axis of the vertical reactor. This arrangement produces, in practice, a reactor comprising two parallel reaction lines, possibly partially communicating with each other. As the two lines thus formed are closed in the same cylindrical body, they allow a considerable constructive simplification with respect to adopting two separate reactors in parallel, especially in the case of high-pressure processes, and avoid resorting to reactors which are highly developed in height when very high flow-rates are necessary and/or big volumes of fluids are to be treated.

In the second advantageous solution, the plane of each single dividing septum is positioned vertically with an angle of from 30° to 90°, more preferably about 90°, with respect to the plane of the septum immediately following or preceding it along the main axis of the reactor. This arrangement with offset dividers induces a transversal re-mixing of the fluids which therefore follow a sinusoidal run in their reascent along the reactor. In this way, a greater mixing of the two phases is obtained.

All the other possible combinations of septa variously orientated with respect to each other, are, however, alternatives included in the scope of the present invention.

One of the advantages of the present invention consists in the flexibility with which the various dividing septa can be constructed and arranged, so as to be able to optimize, also on the basis of the readings during functioning, the fluid-dynamic and kinetic parameters of the reactor.

The dividing septa are made of materials selected on the basis of the process in which the reactor is to be used. They advantageously consist of the same material with which the plates are produced, or a compatible material. The thickness of the dividing septa is not particularly critical, but it is not normally very high as it is not subjected to significant pressure forces. A thickness of a few millimeters, for example, from 1 to 10 mm, preferably from 2 to 5 mm, is sufficient for normal applications.

It has been found that thanks to the present invention, it is possible to maintain the appropriate design and process parameters and contemporaneously lower the height of the reactor even by 40% with respect to that necessary for obtaining the same productivity in the absence of the above dividing septa. The overall diameter of the reactor is proportionally higher, but does not imply a decrease in the efficiency due to the presence of the particular arrangement of the dividing septa.

The enclosed figures provide some illustrative and non-limiting examples, in scale, according to the present invention, with reference to a reactor used for the direct synthesis of urea at high pressures and temperatures. Parts having the same function in different figures are indicated with the same number. For greater clarity, the parts of the apparatuses not necessary for the full understanding and representation of the present invention have been removed from the figures.

The present invention is now described, for illustrative purposes, with particular reference to the enclosed figures, without this representing in any way a limitation of its scope as defined in the claims, also in relation to the solutions equivalent thereto.
Figure 1 schematically represents a perspective view of a vertical reactor according to the present invention, in which the front wall of the cylindrical body is removed to show the most significant internal elements.
Figure 2 schematically represents a more detailed perspective view of a segment of the vertical reactor of figure 1, in which the planes of the dividing septa are offset by 90° with respect to each other.
Figure 3 schematically represents a more detailed perspective view of a segment of the vertical reactor according to the present invention, in which the planes of the dividing septa in adjacent sectors are aligned with each other.
Figure 4 schematically represents a detail of the vertical reactor according to the present invention, in which a dividing septum is positioned between two adjacent plates with seal welding.
Figure 5 schematically represents a detail of the vertical reactor according to the present invention, in which a dividing septum is positioned between two adjacent plates by means of bolting and has openings for fluid communication between the two sections.

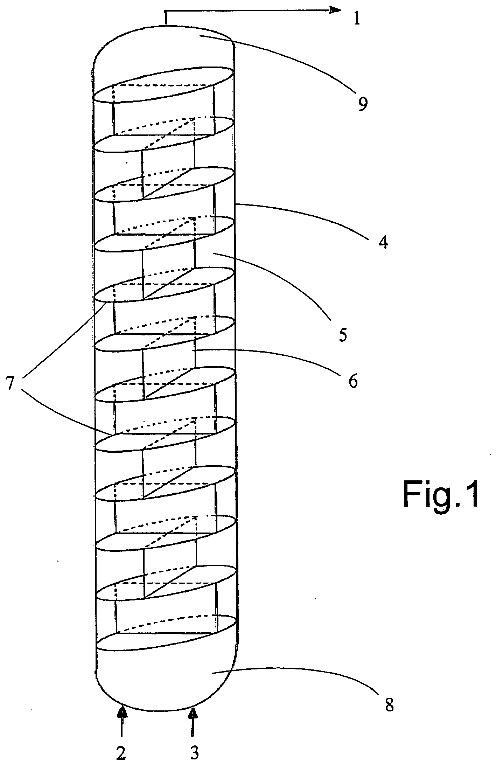

In figure 1, a vertical column reactor can be distinguished, delimited by the force body 4, respectively closed at the ends by the lower cap 8, in which the inlets 2 and 3 of carbon dioxide and the stream comprising liquid ammonia and ammonium carbamate are inserted, and the upper cap 9, in which the reacted mixture is collected, comprising the urea formed in the reactor, sent towards the stripper through line 1 leaving the head. Said line 1 can alternatively leave the reactor at an intermediate height and be connected with a duct arranged vertically inside the apparatus until it reaches an overflow situated in the upper cap of the reactor for the collection of the liquid phase or gas-liquid mixture, according to the cases.

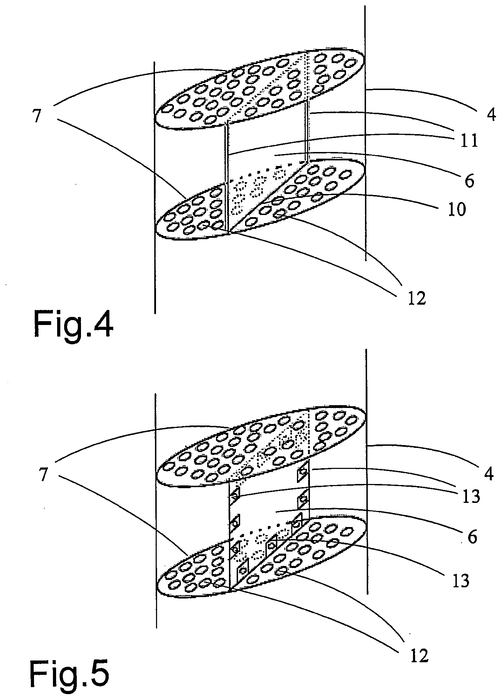

Along the column, a plurality of perforated plates 7 delimits a corresponding number of sectors 5, in which the biphasic reaction mixture flows during functioning. The holes situated in each plate to allow fluid communication between adjacent sectors are not shown in figure 1 but can be distinguished, on the other hand in the subsequent figures 4 and 5. Each sector is divided into two sections substantially having the same volume by means of a dividing septum 6, situated perpendicularly with respect to the adjacent plates and passing through the axis of the reactor. In particular, figure 1 shows dividing septa alternatively arranged with the planes rotated by 90° with respect to each other.

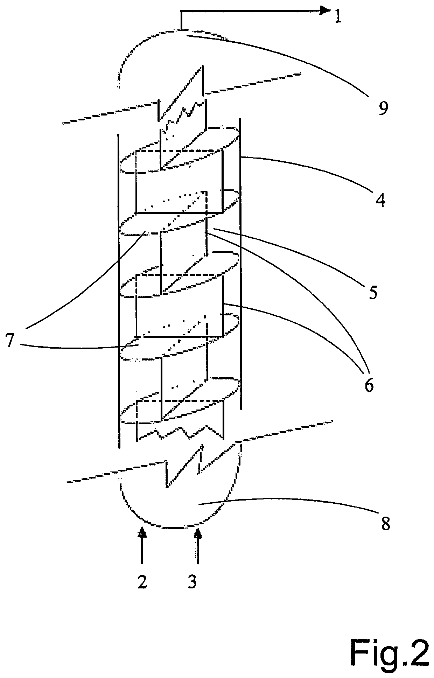

With reference to figure 2, in the section of column represented, essentially the same elements previously described in relation to figure 1 can be distinguished.

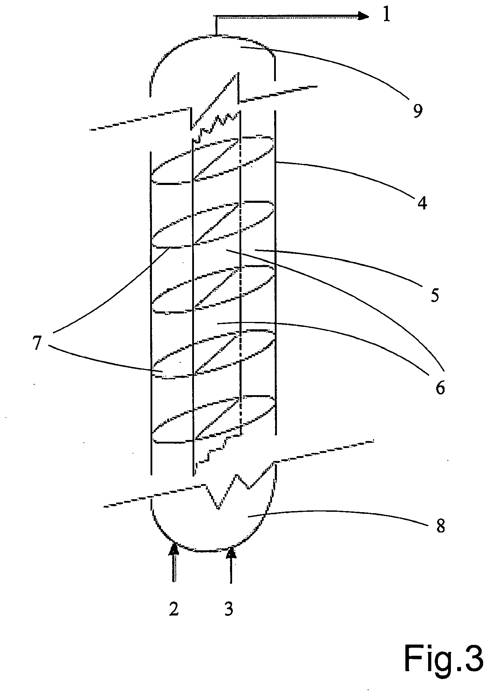

With reference to figure 3, in the section of column represented, essentially the same elements previously described in relation to figure 2 can be distinguished but with the important difference that the dividing septa 6 are all aligned on the same plane.

With reference to figure 4, this illustrates a particular embodiment of the present invention, in which the dividing septum 6 is seal fixed by means of suitable weldings 10 and 11 of its edges, with the surface of the plates 7 and with the internal surface of the lining of the force body 4, respectively.

With reference to figure 5, this illustrates a particular embodiment of the present invention, in which the dividing septum 6 is fixed on the surface of the plates 7 and on the internal surface of the lining of the force body 4, by means of bolted metallic supports 13. This solution facilitates, when necessary, maintenance interventions or modification of the reactor, as it facilitates the removal and substitution of the dividing septa (and possibly the plates, if they are also fixed with bolts). It is also possible to model and fix the dividing septum so that, during functioning, a portion of the reaction mixture migrates from one section to the other of the same sector.

Figures 4 and 5 also schematically represent the holes 12 situated in each plate 7 to allow the flow of the gas/liquid reaction mixture from one sector to the other. These holes can also have forms different from a circular shape, for example, square or rectangular, and can be provided with possible three-dimensional elements such as bells, collars, hinges, grids, suitable for directing or regulating the flow of one or both liquid and gaseous phases, or allowing a more effective mixing. The dimensions of the holes (maximum width) normally range from 1 to 10 cm.

The reactor according to the invention can be produced by applying the normal construction techniques of industrial chemical equipment of this type. In particular, if the reactor is destined for the synthesis or hydrolysis of urea, experts in the field will adopt the most appropriate and widely-used technical solutions for obtaining the equipment operating at high pressures and temperatures, under conditions of high chemical aggressivity of the process fluids, availing, for example, of a force body and steel caps having an adequate thickness internally lined with one or more laminas of a corrosion-resistant metal or alloy, as specified previously. The perforated plates and dividing septa are then arranged inside the reactor, normally operating through the manhole, adequately spaced in relation to the flow characteristics to be obtained. If two dividing intersecting septa are positioned in one or more sectors, the cross-arrangement can be obtained, for example, by welding of the half of a septum, orthogonally on the centre line of each side of the other septum.

The Applicant has also found that a pre-existing vertical plate reactor can be modified for effecting gas/liquid biphasic reactions, so as to obtain, at the end of the intervention, a reactor having the same characteristics as the reactor according to the present invention, with an increased conversion efficiency during functioning. A further object of the present invention therefore relates to a method for modernizing a vertical plate reactor to process biphasic reactions, in addition to the plant in which said reactor is inserted, comprising the positioning of a single dividing septum in at least one sector included between two adjacent plates.

Furthermore, said modernization method can also comprise the removal of one or more plates and repositioning of the remaining plates so as to obtain sectors between adjacent plates having a different height from that of the pre-existing reactor.

With the reactor according to the present invention, or the reactor obtained by means of the above modernization method, it has proved to be particularly advantageous to carry out a process for the production of urea by direct synthesis starting from ammonia and carbon dioxide in a gas-liquid biphasic mixture, at a high pressure and temperature, with the intermediate formation of ammonium carbamate. A further object of the present invention therefore relates to said process in which the formation of urea takes place, either partly or totally, preferably for at least 60%, in the reactor according to the present invention.

With the reactor according to the present invention, the same conversion per passage and process productivity can be obtained as an analogous reactor of the known art not equipped with dividing septa, but with a considerably reduced height with respect to the latter, preferably from 20 to 40% lower. A reactor having a reduced height capable of treating very high volumes of reagent mixture in the absence of dividing septa, on the other hand, is not capable of obtaining a satisfactory conversion efficiency.

A non-limiting example of use of the reactor according to the present invention is now provided for purely illustrative purposes.

### Example

A reactor according to the present invention of the type represented in figures 1 and 2, having the following characteristics, is used in a process for the production of urea with a capacity of 1,750 tons/day:
Internal diameter 265 cm
Height 2,520 cm
Outer wall made of carbon steel, about 10 cm thick
Number of plates 9
Distance between the plates 250 cm

The plates, made of 25/22/2 Cr/Ni/Mo steel having a thickness of 0.8 cm, define a total of 8 cylindrical sectors having a height of about 250 cm, and comprising a quantity of holes of about 3 cm in diameter distributed on their surface with a density of about 400 holes per m².

A vertical dividing septum passing through the axis of the reactor, is positioned in each sector, by bolting, which divides the volume of the sector in half. Said septum does not comprise holes and consists of a 25/22/2 Cr/Ni/Mo steel lamina having a thickness of 0.8 cm. As can be well seen in figure 2, the plane of each dividing septum is rotated by 90° along the axis of the reactor with respect to each of the septa immediately adjacent.

54,200 kg/h of a gaseous stream of CO₂ containing 750 kg/h of inert products, are introduced, through line 2, into the reactor, having a temperature of 120°C and a pressure of 16 MPa. A liquid stream containing ammonium carbamate and ammonia in excess enters the reactor, through line 3, at a temperature of 135°C and a pressure of 16 MPa, having the following composition:

| | | |
|---|---|---|
| NH₃ | 114,246 | kg/h, |
| CO₂ | 31,467 | kg/h, |
| H₂O | 23,660 | kg/h |

wherein the ammonium carbamate is expressed as CO₂ and the quantity of ammonia comprises both free ammonia and that bound to the ammonium carbamate. The reaction mixture which is formed at the bottom of the reactor rises up through the various plates and the sections in which the sectors are divided between one plate and another. With each plate passage, the reaction mixture of one section is partially mixed with that of the adjacent section in the same sector, favouring the homogeneous attainment of equilibrium. The flow of material, under these conditions, is such as to prevent the turbulance from producing a significant back-mixing. At the end, a mixed gas/liquid stream is taken from the head of the reactor, through line 1, at 190°C and 15.5 MPa, having the following composition:

| | | |
|---|---|---|
| NH₃ | 73,608 | kg/h |
| CO₂ | 31,609 | kg/h |
| Urea | 73,187 | kg/h |
| H₂O | 44,001 | kg/h |
| Inert products | 750 | kg/h |

wherein the CO₂ is essentially present in saline form, as ammonium carbamate. This stream is sent to the separation and purification sections of urea, operating according to the known techniques, from which an aqueous stream of ammonium carbamate and ammonia is recycled to the reactor through said line 3, after the addition of make-up ammonia.

The conversion per passage of CO₂ to urea, calculated as molar ratio percentage between urea produced and total CO₂ supplied by the lines 2 and 3, is equal to 63%.

A reactor of the traditional type, in order to obtain the same conversion to urea, with the same flow-rates as those illustrated above for producing 1,750 tons per day of urea, normally has a diameter of 210 cm and a height of 4,000 cm.

The great advantage of the reactor according to the invention is that, with the same conversion efficiency, it becomes easier to construct and transport, and the maintenance is facilitated, once it has started operating.

For comparative purposes, it was also evaluated, through modellistic calculations, that a reactor having the same dimensions as that described above, but not equipped with the above dividing septa, if operating with the same productivity regime of 1,750 tons per day, would produce urea with a conversion per passage of CO₂ of 57%.

Embodiments of the present invention, different from those described above, can be obtained by an expert in the field, adapting to the various applicative requirements, which represent obvious variants, in any case included in the scope of the following claims.

## Claims

1. A vertical reactor for the production of urea by direct synthesis from ammonia and carbon dioxide in a gas-liquid biphasic mixture, comprising:
a) a hollow structure delimited by an outer wall (4) having a substantially cylindrical form, closed at the ends by substantially hemispherical caps (8, 9), comprising openings for the inlet and outlet of the process fluids so as to have gas and liquid phases flowing co-currently within the reactor; a lower cap (8) having inlets (2, 3) of carbon dioxide and a stream comprising liquid ammonia and ammonium carbamate respectively; a reacted mixture comprising urea formed in the reactor being collected in an upper cap (9) and sent to a line (1) leaving the reactor either from the upper cap or at an intermediate height via a duct arranged vertically inside the apparatus and connected to an overflow situated in the upper cap (9);
b) a plurality of superimposed perforated plates (7), extending horizontally inside the structure as far as the internal surface of the cylindrical wall (4) and suitably spaced along the vertical axis, so that between each pair of adjacent plates there is a sector (5) in fluid communication with the sector respectively positioned above and/or below the same;
**characterized in that** at least one sector (5) comprises one single substantially planar dividing septum (6) situated between two adjacent plates and perpendicular to these and fixed on the surface of each of the plates (7) and on the internal surface of the lining of the outer wall (4), so as to divide the respective sector into two sections whose volumes are in a ratio between each other of 1/3 to 3/1, preferably from 0.95 to 1.05, more preferably equal to 1.

2. The vertical reactor according to claim 1, wherein at least 2 sectors defined by adjacent plates comprises at least one dividing septum.

3. The vertical reactor according to claim 1, wherein at least 90% of the sectors defined by adjacent plates comprises at least one dividing septum.

4. The vertical reactor according to any of the previous claims, wherein the number of plates varies from 5 to 40, preferably from 10 to 20.

5. The vertical reactor according to any of the previous claims, wherein the distance between adjacent plates ranges from 50 to 400 cm.

6. The vertical reactor according to claim 5, wherein the distance between adjacent plates is almost constant and ranges from 150 to 300 cm.

7. The vertical reactor according to any of the previous claims, wherein the plates and dividing septa consist of a metal or alloy selected from titanium, zirconium, AISI 316L steel, austenoferritic steels, INOX 25/22/2 Cr/Ni/Mo steel.

8. The vertical reactor according to any of the previous claims, wherein the average density of the holes in a perforated plate ranges from 100 to 1000 holes per m².

9. The vertical reactor according to any of the previous claims, wherein all the single dividing septa are substantially aligned on a single geometrical plane containing the main axis of the vertical reactor.

10. The vertical reactor according to any of the previous claims from 1 to 9, wherein the plane of each single dividing septum is positioned vertically with an angle of from 30° to 90° with respect to the plane of the septum immediately following or preceding it along the main axis of the reactor.

11. The vertical reactor according to any of the previous claims, wherein said dividing septum is seal fixed by weldings of its edges.

12. The vertical reactor according to any of the previous claims from 1 to 10, wherein said dividing septum is fixed by means of bolted metallic supports.

13. A method for improving a pre-existing vertical reactor, of the plate type, for the production of urea by direct synthesis from ammonia and carbon dioxide in a gas-liquid biphasic mixture, the reactor to be improved comprising:
a) a hollow structure delimited by an outer wall having a substantially cylindrical form, closed at the ends by substantially hemispherical caps (8, 9), comprising openings for the inlet and outlet of the process fluids configured and arranged so as to have gas and liquid phases flowing co-currently within the reactor; a lower cap (8) having inlets (2, 3) of carbon dioxide and a stream comprising liquid ammonia and ammonium carbamate respectively; a reacted mixture comprising urea formed in the reactor being collected in an upper cap (9) and sent to a line (1) leaving the reactor either from the upper cap or at an intermediate height via a duct arranged vertically inside the apparatus and connected to an overflow situated in the upper cap (9);
b) a plurality of superimposed perforated plates, extending horizontally inside the structure as far as the internal surface of the cylindrical wall and suitably spaced along the vertical axis, so that between each pair of adjacent plates a sector is obtained which is in fluid communication with the sector positioned above and/or below the same, respectively;
the method being **characterized in that** the reactor is improved by positioning one single substantially planar dividing septum in at least one, preferably in over 90%, more preferably in all of said sectors, said septum being arranged between two adjacent plates and perpendicular to these and fixed on the surface of each of the plates (7) and on the internal surface of the lining of the outer wall (4), so as to divide the respective sector into two sections whose volumes are in a ratio between each other of 1/3 to 3/1, preferably from 0.95 to 1.05, more preferably equal to 1.

14. A process for the production of urea by means of direct synthesis starting from ammonia and carbon dioxide with the intermediate formation of ammonium carbamate, **characterized in that** the synthesis reaction is carried out in a gas-liquid biphasic mixture, at a high pressure and temperature, in a vertical reactor according to any of the previous claims from 1 to 12.

## Patentansprüche

1. Vertikalreaktor für die Herstellung von Harnstoff durch Direktsynthese von Ammoniak und Kohlenstoffdioxid in einem zweiphasigen Gas-Flüssigkeitsgemisch, umfassend:
a) eine Hohlstruktur, die durch eine Außenwand (4) begrenzt ist, die eine im Wesentlichen zylindrische Form aufweist, die an den Enden durch im Wesentlichen halbkugelförmige Kappen (8, 9) verschlossen ist, die Öffnungen für den Einlass und Auslass von Prozessfluiden umfassen, sodass gasförmige und flüssige Phasen gleichzeitig innerhalb des Reaktors strömen; wobei eine untere Kappe (8) Einlässe (2, 3) von jeweils Kohlenstoffdioxid und einen Strom umfassend flüssiges Ammoniak und Ammoniumcarbamat aufweist; wobei ein reagiertes Gemisch umfasst, dass in dem Reaktor gebildeter Harnstoff in einer oberen Kappe (9) gesammelt und zu einer Leitung (1) gesendet wird, die den Reaktor entweder von der oberen Kappe oder bei einer mittleren Höhe über ein Rohr verlässt, das vertikal innerhalb der Einrichtung angeordnet und mit einem Überlauf verbunden ist, der sich in der oberen Kappe (9) befindet;
b) eine Vielzahl von übereinander gelagerten Lochplatten (7), die sich horizontal innerhalb der Struktur bis zu der inneren Oberfläche der zylindrischen Wand (4) erstrecken und angemessen entlang der vertikalen Achse beabstandet sind, sodass sich zwischen jedem Paar angrenzender Platten ein Sektor (5) in Fluidkommunikation mit dem jeweils darüber und/oder darunter positionierten Sektor befindet;
**dadurch gekennzeichnet, dass** mindestens ein Sektor (5) eine einzelne, im Wesentlichen ebene unterteilende Trennwand (6) umfasst, die sich zwischen zwei angrenzenden Platten und senkrecht zu diesen befindet und an der Oberfläche jeder der Platten (7) und an der inneren Oberfläche der Auskleidung der Außenwand (4) befestigt ist, um so den jeweiligen Sektor in zwei Abschnitte zu unterteilen, deren Volumen in einem Verhältnis von 1/3 bis 3/1, vorzugsweise von 0,95 bis 1,05, weiter bevorzugt gleich 1 zueinander liegen.

2. Vertikalreaktor nach Anspruch 1, wobei mindestens 2 Sektoren, die durch angrenzende Platten bestimmt sind, mindestens eine unterteilende Trennwand umfassen.

3. Vertikalreaktor nach Anspruch 1, wobei mindestens 90 % der Sektoren, die durch angrenzende Platten bestimmt sind, mindestens eine unterteilende Trennwand umfassen.

4. Vertikalreaktor nach einem der vorhergehenden Ansprüche, wobei die Anzahl von Platten von 5 bis 40, vorzugsweise von 10 bis 20 variiert.

5. Vertikalreaktor nach einem der vorhergehenden Ansprüche, wobei der Abstand zwischen angrenzenden Platten im Bereich von 50 bis 400 cm liegt.

6. Vertikalreaktor nach Anspruch 5, wobei der Abstand zwischen angrenzenden Platten beinahe gleichmäßig ist und im Bereich von 150 bis 300 cm liegt.

7. Vertikalreaktor nach einem der vorhergehenden Ansprüche, wobei die Platten und unterteilenden Trennwände aus einem Metall oder einer Legierung bestehen, die ausgewählt ist aus Titan, Zirkon, AISI-316L-Stahl, austenitischferritischen Stählen, INOX-25/22/2-Cr/Ni/Mo-Stahl.

8. Vertikalreaktor nach einem der vorhergehenden Ansprüche, wobei die durchschnittliche Dichte der Löcher in einer Lochplatte im Bereich von 100 bis 1000 Löcher pro m² liegt.

9. Vertikalreaktor nach einem der vorhergehenden Ansprüche, wobei alle einzelnen unterteilenden Trennwände im Wesentlichen auf einer einzelnen geometrischen Ebene angeordnet sind, die die Hauptachse des Vertikalreaktors enthält.

10. Vertikalreaktor nach einem der vorhergehenden Ansprüche 1 bis 9, wobei die Ebene jeder einzelnen unterteilenden Trennwand vertikal mit einem Winkel von 30° bis 90° in Bezug auf die Ebene der ihr entlang der Hauptachse des Reaktors unmittelbar folgenden oder vorausgehenden Trennwand positioniert ist.

11. Vertikalreaktor nach einem der vorhergehenden Ansprüche, wobei die unterteilende Trennwand durch Verschweißungen ihrer Kanten abdichtend befestigt ist.

12. Vertikalreaktor nach einem der vorhergehenden Ansprüche 1 bis 10, wobei die unterteilende Trennwand mittels verschraubter metallischer Stützen befestigt ist.

13. Verfahren zum Verbessern eines bereits bestehenden Vertikalreaktors des Plattentyps zur Herstellung von Harnstoff durch Direktsynthese von Ammoniak und Kohlenstoffdioxid in einem zweiphasigen Gas-Flüssigkeitsgemisch, wobei der zu verbessernde Reaktor Folgendes umfasst:
a) eine Hohlstruktur, die durch eine Außenwand begrenzt ist, die eine im Wesentlichen zylindrische Form aufweist, die an den Enden durch im Wesentlichen halbkugelförmige Kappen (8, 9) verschlossen ist, die Öffnungen für den Einlass und Auslass von Prozessfluiden umfassen, die ausgestaltet und angeordnet sind, sodass gasförmige und flüssige Phasen gleichzeitig innerhalb des Reaktors strömen; wobei eine untere Kappe (8) Einlässe (2, 3) von jeweils Kohlenstoffdioxid und einen Strom umfassend flüssiges Ammoniak und Ammoniumcarbamat aufweist; wobei ein reagiertes Gemisch umfasst, dass in dem Reaktor gebildeter Harnstoff in einer oberen Kappe (9) gesammelt und zu einer Leitung (1) gesendet wird, die den Reaktor entweder von der oberen Kappe oder bei einer mittleren Höhe über ein Rohr verlässt, das vertikal innerhalb der Einrichtung angeordnet und mit einem Überlauf verbunden ist, der sich in der oberen Kappe (9) befindet;
b) eine Vielzahl von übereinander gelagerten Lochplatten, die sich horizontal innerhalb der Struktur bis zu der inneren Oberfläche der zylindrischen Wand erstrecken und angemessen entlang der vertikalen Achse beabstandet sind, sodass zwischen jedem Paar angrenzender Platten ein Sektor erhalten wird, der sich in Fluidkommunikation mit dem jeweils darüber und/oder darunter positionierten Sektor befindet;
wobei das Verfahren **dadurch gekennzeichnet ist, dass** der Reaktor durch Positionieren einer einzelnen im Wesentlichen ebenen unterteilenden Trennwand in mindestens einem, vorzugsweise in über 90 %, weiter bevorzugt in allen der Sektoren verbessert wird, wobei die Trennwand zwischen zwei angrenzenden Platten und senkrecht zu ihnen angeordnet ist und an der Oberfläche jeder der Platten (7) und an der inneren Oberfläche der Auskleidung der Außenwand (4) befestigt ist, um so den jeweiligen Sektor in zwei Abschnitte zu unterteilen, deren Volumen in einem Verhältnis von 1/3 bis 3/1, vorzugsweise von 0,95 bis 1,05, weiter bevorzugt gleich 1 zueinander liegen.

14. Prozess zur Herstellung von Harnstoff mittels Direktsynthese ausgehend von Ammoniak und Kohlenstoffdioxid mit der Zwischenproduktbildung von Ammoniumcarbamat, **dadurch gekennzeichnet, dass** die Synthesereaktion in einem zweiphasigen Gas-Flüssigkeitsgemisch unter hohem Druck und hoher Temperatur in einem Vertikalreaktor nach einem der vorhergehenden Ansprüche 1 bis 12 stattfindet.

## Revendications

1. Réacteur vertical pour la production d'urée par synthèse directe à partir d'ammoniac et de dioxyde de carbone dans un mélange biphasique gaz-liquide, comprenant :
a) une structure creuse délimitée par une paroi (4) extérieure ayant une forme sensiblement cylindrique, fermée aux extrémités par des capuchons (8, 9) sensiblement hémisphériques, comprenant des ouvertures pour l'admission et l'évacuation des fluides de traitement de façon à avoir des phases gazeuse et liquide s'écoulant à co-courant dans le réacteur ; un capuchon (8) inférieur ayant des admissions (2, 3) de dioxyde de carbone et d'un flux comprenant de l'ammoniac liquide et du carbamate d'ammonium respectivement ; un mélange ayant réagi comprenant de l'urée formée dans le réacteur étant collecté dans un capuchon (9) supérieur et envoyé vers une conduite (1) quittant le réacteur soit depuis le capuchon supérieur soit à une hauteur intermédiaire via un conduit agencé verticalement à l'intérieur de l'appareil et relié à un trop-plein situé dans le capuchon (9) supérieur ;
b) une pluralité de plaques (7) perforées superposées, s'étendant horizontalement à l'intérieur de la structure aussi loin que la surface interne de la paroi (4) cylindrique et espacées de manière appropriée le long de l'axe vertical, de façon à ce qu'entre chaque paire de plaques adjacentes il y ait un secteur (5) en communication de fluide avec le secteur respectivement positionné au-dessus et/ou en dessous de celui-ci ;
**caractérisé en ce qu'**au moins un secteur (5) comprend un septum de division (6) sensiblement plat unique situé entre deux plaques adjacentes, perpendiculaire à celles-ci et fixé sur la surface de chacune des plaques (7) et sur la surface interne du revêtement de la paroi (4) extérieure, de façon à diviser le secteur respectif en deux sections dont les volumes ont un rapport l'un avec l'autre de 1/3 à 3/1, de préférence de 0,95 à 1,05, de manière davantage préférée égal à 1.

2. Réacteur vertical selon la revendication 1, dans lequel au moins 2 secteurs définis par des plaques adjacentes comprennent au moins un septum de division.

3. Réacteur vertical selon la revendication 1, dans lequel au moins 90 % des secteurs définis par des plaques adjacentes comprennent au moins un septum de division.

4. Réacteur vertical selon l'une quelconque des revendications précédentes, dans lequel le nombre de plaques varie de 5 à 40, de préférence de 10 à 20.

5. Réacteur vertical selon l'une quelconque des revendications précédentes, dans lequel la distance entre des plaques adjacentes va de 50 à 400 cm.

6. Réacteur vertical selon la revendication 5, dans lequel la distance entre des plaques adjacentes est presque constante et va de 150 à 300 cm.

7. Réacteur vertical selon l'une quelconque des revendications précédentes, dans lequel les plaques et septums de division consistent en un métal ou un alliage sélectionnés parmi du titane, du zirconium, de l'acier AISI 316L, des aciers austéno-ferritiques, de l'acier Cr/Ni/Mo INOX 25/22/2.

8. Réacteur vertical selon l'une quelconque des revendications précédentes, dans lequel la densité moyenne des trous dans une plaque perforée va de 100 à 1000 trous par m².

9. Réacteur vertical selon l'une quelconque des revendications précédentes, dans lequel tous les septums de division uniques sont sensiblement alignés sur un plan géométrique unique contenant l'axe principal du réacteur vertical.

10. Réacteur vertical selon l'une quelconque des revendications 1 à 9 précédentes, dans lequel le plan de chaque septum de division unique est positionné verticalement avec un angle de 30° à 90° par rapport au plan du septum le suivant ou le précédant immédiatement le long de l'axe principal du réacteur.

11. Réacteur vertical selon l'une quelconque des revendications précédentes, dans lequel ledit septum de division est fixé et scellé par des soudages de ses bords.

12. Réacteur vertical selon l'une quelconque des revendications 1 à 10 précédentes, dans lequel ledit septum de division est fixé au moyen de supports métalliques boulonnés.

13. Procédé pour améliorer un réacteur vertical préexistant, du type à plaques, pour la production d'urée par synthèse directe à partir d'ammoniac et de dioxyde de carbone dans un mélange biphasique gaz-liquide, le réacteur devant être amélioré comprenant :
a) une structure creuse délimitée par une paroi extérieure ayant une forme sensiblement cylindrique, fermée aux extrémités par des capuchons (8, 9) sensiblement hémisphériques, comprenant des ouvertures pour l'admission et l'évacuation des fluides de traitement configurées et agencées de façon à avoir des phases gazeuse et liquide s'écoulant à co-courant dans le réacteur; un capuchon (8) inférieur ayant des admissions (2, 3) de dioxyde de carbone et d'un flux comprenant de l'ammoniac liquide et du carbamate d'ammonium respectivement; un mélange ayant réagi comprenant de l'urée formée dans le réacteur étant collecté dans un capuchon (9) supérieur et envoyé vers une conduite (1) quittant le réacteur soit depuis le capuchon supérieur soit à une hauteur intermédiaire via un conduit agencé verticalement à l'intérieur de l'appareil et relié à un trop-plein situé dans le capuchon (9) supérieur ;
b) une pluralité de plaques perforées superposées, s'étendant horizontalement à l'intérieur de la structure aussi loin que la surface interne de la paroi cylindrique et espacées de manière appropriée le long de l'axe vertical, de façon à ce qu'entre chaque paire de plaques adjacentes un secteur soit obtenu qui est en communication de fluide avec le secteur positionné au-dessus et/ou en dessous de celui-ci, respectivement;
le procédé étant **caractérisé en ce que** le réacteur est amélioré en positionnant un septum de division sensiblement plat unique dans au moins un, de préférence dans plus de 90 %, de manière davantage préférée dans tous lesdits secteurs, ledit septum étant agencé entre deux plaques adjacentes et perpendiculaire à celles-ci et fixé sur la surface de chacune des plaques (7) et sur la surface interne du revêtement de la paroi (4) extérieure, de façon à diviser le secteur respectif en deux sections dont les volumes ont un rapport l'un avec l'autre de 1/3 à 3/1, de préférence de 0,95 à 1,05, de manière davantage préférée égal à 1.

14. Procédé pour la production d'urée au moyen d'une synthèse directe partant de l'ammoniac et du dioxyde de carbone avec la formation intermédiaire de carbamate d'ammonium, **caractérisé en ce que** la réaction de synthèse est mise en œuvre dans un mélange biphasique gaz-liquide, à une pression et une température élevées, dans un réacteur vertical selon l'une quelconque des revendications 1 à 12 précédentes.
